# EUROPEAN PATENT SPECIFICATION

(11) **EP 2 274 018 B1**
(45) Date of publication and mention of the grant of the patent: **18.12.2013**
(21) Application number: 08735873.5
(22) Date of filing: 07.04.2008
(51) Int. Cl.: A61K 47/48, A61K 51/06, A61P 35/00

(54) **Modified hydroxypolymer conjugates with bone-seeking and tumor-killing moieties**
Modifizierte Hydroxylpolymer-Konjugate mit Knochengewebe-gerichteten und Tumor-vernichtenden Gruppen
Conjugués d'hydroxypolymères modifiés avec des groupes ostéotropiques et de destructeurs de tumeur

(43) Date of publication of application: 19.01.2011
(73) Proprietor: Dextech Medical AB, 75 104 Uppsala (SE)
(72) Inventor: HOLMBERG, Anders, S-75106 Uppsala (SE)
(74) Representative: Sahlin, Jonna Elisabeth
(86) International application number: PCT/EP2008/054139
(87) International publication number: WO 2009/124580

(56) References cited:
- WO-A-00/21571
- WO-A-00/23023
- WO-A-97/45401
- WO-A-02/056861
- WO-A-2005/105058
- US-A- 5 011 913
- US-A1- 2005 090 553

## Description

### Technical Field of Invention

The present invention is related to a modified hydroxypolymer conjugate substituted with bone seeking and tumor killing moieties including anti-osteoclastic activity. The modified hydroxypolymer conjugate is useful as a medicine for treating bone cancer, osteoporosis, etc. The hydroxypolymer dextran conjugate is used for treating metastatic bone lesions in refractory prostate cancer. The invention is also related to a method for preparation of said hydroxypolymer conjugate as well as its use.

### Background of Invention

Hydroxypolymers are a group of sugar polymers, which are provided with a multitude of hydroxy groups, which are conveniently substituted with different moieties of effective compounds. Dextran is one of most frequently used hydroxypolymers in pharmacetucial industry with established pharmaceutical uses, e.g. for preventing hypovolemic chock, preventing embolism and improving microcirculation. Dextran and its non-toxic properties and high tolerability is very well documented (AS Segal, 1964 In: Modem medical monographs, ed. Wright, IS, Green & Stratton, NY, London, pp 5-17. Therefore, it is often used as an example of pharmaceutically applicable hydroxypolymers.

In the International patent application PCT/EP2008/052714 it is demonstrated that although guanidine compounds and dextrans, separately used have almost no toxic effect on tumor cells, guanidine dextran conjugates demonstrated an antitumor activity that was similar or at some dosages even higher than that of convention antitumor drugs, like Adriamycin^{®}

It is common knowledge that different types of tumor require different types of antitumor drugs and treatment modalities. Bone metastasis is common in advanced cancer. The incidence of bone metastasis is especially high in advanced breast and prostate cancer. The majority of these patients, approximately 80%, have extensive skelletal metastasis. Skelletal pain is the most common cancer related pain and is often intense with a profound negative effect on the quality of life. Standard treatments include combinations of analgesics and chemotherapeutic drugs, radionuclide treatment, localised external beam radiation, and local surgery. Some patients experience long term pain palliation, however, many continue with pain in spite of therapy. An investigation in the US showed that about 25% continued with severe uncontrolled pain in spite of therapy. In view of the percentages shown above it is evident that there is great need of new and more effective medicine for treating skeletal cancer i.e. bone metastasis (Gralow et al., Journal of Pain and Symptom Management, 33(4), 2007; G Owens, Am J Manag Care. 13,290-308, 2007; Hoesl et al., Urol Int, 76:97-105, 2006; , Márquez et al., Anticancer Res, May-Jun;24(3a):1347-51, 2004; , Lambronoudaki et al., Ann. N.Y. Acad. Sci. 1092;397-402, 2006); Daubiné et al., J Natl Cancer Inst 99, 322-330, 2007; Larsson et al., Anticancer Res, 1989, 9:1111-1119, 1989).

### Summary of Invention

The present invention is related to modified hydroxypolymer conjugates for manufacturing of a pharmaceutical for the treatment of tumors located in the skeleton i.e. bone metastasis and for the treatment of osteoporosis. The modified hydroxypolymer conjugates are conjugates, wherein biphosphonate and guanidine compounds having at least one free amino group are covalently coupled to the activated hydroxyl groups of a hydroxypolymer. The hydroxypolymer is a dextran, which has a molecular weight of 10³-10⁶ Daltons and is activated. The preferred biphosphonates compounds comprise neridronate or alendronate and the guanidine compounds are agmatine or aminoguanidine.

### Description of Drawings

Figure 1 shows the results of a fluorometric cytotoxicity assay performed as described in Larsson and Nygren (Larson et al, Anticancer Res, 1989, 9:1111-1119). Dextran-Guanidine-Biphosphonate (DGB) was compared with Zoledronic acid (Zometa®), at concentrations 0.4, 0.9, 1.75, 3.5, 7 and 25 µM. In Figure 1 y axe shows % cell survival of the cell line PC3 (prostata cancer) and the x axe shows the molarity of the compared test substances.
**Figure 2** shows the results of the same fluorometric cytotoxicity assay described in Figure 1. In Figure 2 the y axe shows % cell survival of the cell line MDA453 (breast cancer) and the x axe shows the molarity of the compared test substances.

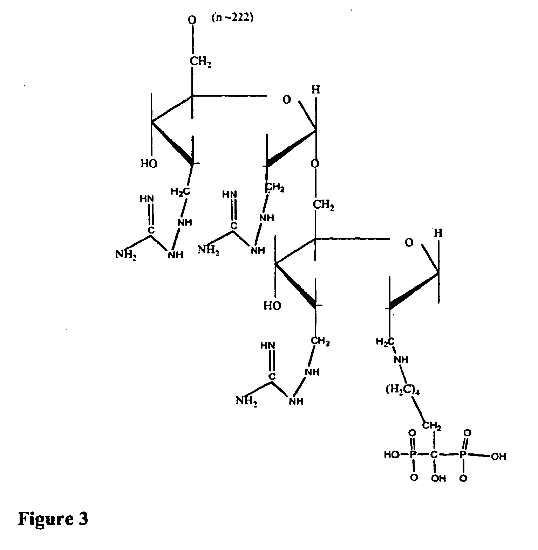

**Figure 3** depicts the structure of a typical guanidine-dextran-biphosphonate conjugate wherein the glucose units of a dextran molecule are substituted with guanidine groups and biphosphonate group.
**Figure 4** is a gamma image (ant) of a lesion in the right shoulder 6,5 hours post injection (hpi) treated with a guanidine-dextran-biphosphonate conjugate.
**Figure 5** is a gamma image (ant) of a lesion in the right shoulder 24 hpi treated with a guanidine-dextran-biphosphonate conjugate.
**Figure 6** is a gamma image (ant) of a lesion in the right femur 6 hpi treated with a guanidine-dextran-biphosphonate conjugate.
**Figure 7** is a gamma image (ant) of a lesion in the right knee 6 hpi treated with a guanidine-dextran-biphosphonate conjugate.
**Figure 8** is a gamma image (ant) of a lesion in the right shoulder 6,5 hpi treated with a guanidine-dextran-biphosphonate conjugate.

### Detailed Description of Invention

In the International patent application PCT/EP2008/052714, the inventors demonstrated that certain guanidine compounds, having very low intrinsic toxicity, when covalently conjugated to a non-toxic hydroxyl polymer, such as dextran, have a high tumor cell killing efficacy. The tumor cell killing efficacy of dextran guanidine was shown to be equal to established anthracycline anticancer pharmaceuticals such as Farmorubicine® and Adriamycine® or at some concentrations higher than established anticancer drugs. It appears probable that due to the harmless nature of the constituents of said polymer-guanidine conjugates, the handling of such conjugates i.e. for medical staff and the treatment connected side-effects for the patients can be expected to be favourable in comparison to conventional chemotherapeutic anti-cancer pharmaceuticals.

Targeting or tumor cell selectivity is believed to be achieved through the increased metabolic needs of the fast dividing tumor cells, which require amino-5-guanidinopentanoic acid and structurally related guanidine compounds, and other constituents for polyamine formation (D Scott Lind, Am Soc Nutr Sci, 2004, J Nutr, 134:2837-2841; E Wayne, Turk J Med Sci, 2003, 33, 195-205). Cellular uptake of said guanidine compounds is mediated principally via the Na+ -independent basic amino acid transport system y+ (Cendan et al, Ann Surg Oncol, 1995, 2:257-265).

The present invention is a modified hydroxypolymer conjugate, i.e. a modified dextran substituted with both bone seeking biphosphonate groups and tumor cell killing guanidine groups. The modified hydroxypolymer conjugates is a tumor cell killing dextran-guanidine-biphosphonate compound having anti-osteoclastic activity. The modified hydroxypolymer conjugate is a hydroxypolymer, such as a dextran substituted with guanidine compounds, which have at least one free amino group and which are covalently coupled to activated hydroxyl groups of the hydroxypolymer moiety;
and which are agmatine or aminoguanidine. The hydroxypolymer moiety is a dextran, which has a molecular weight in the range 10³-10⁶ Daltons and which is activated before being substituted with said guanidine compounds.

The modified hydroxypolymer conjugate may further comprise covalently coupled functional groups, including radio-nuclides, therapeutic compounds, anticancer drugs, targeting agents, etc.

The modified hydroxypolymer conjugate is a guanidine-dextran conjugate, wherein the dextran preferably has a molecular weight in the range of 10³-10⁶ Daltons and wherein at least 15%, preferably 20% of the glucose moieties of the hydroxypolymer compound, e.g. dextran, are substituted with guanidine compounds having at least one free amino side group, and preferably at least 10%, preferably 15% of said glucose moieties of the hydroxypolymer compound, e.g. dextran are substituted with biphosphonate compounds. Said guanidine compounds, agmatine or aminoguanidine, are covalently coupled to activated hydroxyl groups of the said hydroxypolymer compound.

The biphosphonate compounds are preferably alendronate, neridronate or other similar biphosphonate compounds. The hydroxypolymer conjugates may further comprise covalently coupled functional groups, including radio-nuclides, therapeutic compounds, anticancer drugs, targeting agents, etc.

The present invention is also related to the use of said modified hydroxypolymer conjugates as medicines, particularly for manufacturing medicines for treating bone tumors i.e. metastatic bone lesions, and osteoporosis,

The medicines are tumor killing compositions and with ant-osteoclastic activity and they comprise the modified hydroxypolymer conjugate and at least one pharmaceutically acceptable adjuvant. The medicines or tumor killing compositions are locally, intravesically, regiolocally, peritoneally, intratumorally, systemically or intravenously administrable. The medicines may further comprise covalently coupled functional groups, including radio-nuclides, therapeutic compounds, anticancer drugs, targeting agents, etc.

The modified hydroxypolymer conjugate of the invention is a guanidine-dextran-biphosphonate compound for preferably treating metastatic bone lesions.

The present invention also provides methods for producing the modified hydroxypolymer conjugate. The hydroxypolymer is activated by an oxidative reaction, wherein a periodate salt is added to an aqueous solution comprising said hydroxypolymer with subsequent addition of concentrated sulphuric acid and by ending the reaction by adding ethylene glycol. The hydroxypolymer, which has been activated as described above, is subsequently purified by gel filtration.

A guanidine compound and a biphosphonate compound are sequentially added to the solution comprising the purified activated hydroxypolymer. The order of addition is interchangeable. In order to obtain better control of the substitution degree in or the percentage of the guanidine or biphosphonate compounds present in the modified hydroxypolymer conjugate the hydroxypolymer is first conjugated with the compound that is intended to be present in larger amounts followed by the other compound, which is intended to be present in smaller amounts. If it is desired that biphosphonate compound should be present in smaller amounts it is added secondly, and the guanidine firstly. If it is desired that guanidine compound should be present in smaller amounts it is added secondly, and the biphosphonate group firstly. It is naturally, possible to add both conjugates simultaneously, but the result in that case is more haphazard or less controllable.

Generally, it is desirable that the modified hydroxypolymer conjugate of the present invention should comprise 30 mol of the guanidine compound and 30 mol of the biphosphonate compound per mol hydroxypolymer or dextran. Naturally, the modified hydroxypolymer conjugates may comprise various amounts of randomly located guanidine and biphosphonate depending on the amounts of starting material used. In the present invention the modified hydroxypolymer conjugates should contain at least 10 mol biphosphonate, preferably 20 mol biphosphonate or most preferably 25 mol biphosphonate and at least 25 mole guanidine, preferably 30 mol guanidine or most preferably 35 mol guanidine per mol hydroxypolymer compound. The amount of the biphosphonate may be greater than 30 mol per mol hydroxypolymer. In that case the amount of guanidine should be smaller than 30 mol. Alternatively, the amount of guanidine may be more than 30 to 35 mol per mol hydroxypolymer compound, in which case the amount of biphosphonate should be smaller than 30 mol.

The dextran polymer is substituted with side groups of guanidine. The guanidine groups are covalently coupled to said dextran polymer via free amino side groups of the guanidine compounds. The substitution is preceded by activating said dextran polymer through oxidation, which enables the reaction with said free amino side groups forming bonds of guanidine compounds with subsequent reduction obtain stable amine bonds (Matsunaga et al, Nucl Med Biol. 2005, 32, 279-285).

The modified hydroxypolymer conjugate is provided with a functional group that is biphosphonate. Thus, the modified hydroxypolymer conjugate is a dextran-guanidine-biphosphonate conjugate, wherein the biphosphonate group is preferably a neridronate or alendronate group.

The modified hydroxypolymer conjugate dextran-guanidine-biphosphonate is particularly useful for treating metastatic bone lesions in advanced prostate cancer, i.e. hormone refractory prostate cancer (HRPC), and may affect osteoclastic activity. The dextran-guanidine-biphosphonate conjugate includes as targeting agents the bone seeking biphosphonate moieties, e.g. neridronatete or alendronate and as tumor cell killing moieties the guanidine groups agmatine or aminoguanidine. Suitable biphosphonates can be found among a group of pharmaceuticals which have high affinity to remodelling bone.

Other functional groups that may be coupled additionally to said guanidine hydroxypolymer conjugates compounds include therapeutic drugs, radionuclides, such as. Tc-99m, I-131, Y-90, Re-188, Sm-153, hormones, hormone antagonists, such as Tamoxifen, peptides, such as somatostatin, toxins, monoclonal antibodies or fragments thereof, free radical scavengers, such as amifostine or proteins and targeting agents. Coupling of such functional groups is achieved directly or via bifunctional chelates that have been coupled to the hydroxypolymer prior to the inclusion of functional groups.

The modified hydroxypolymer conjugates of the present invention is preferably administered locally, e.g. by intra-vesical administration, regiolocally, e.g. by administration to the peritoneal cavity, intratumorally, i.e. by injection directly into a solid tumor, or systemically, i.e. by intra-venous administration. All these routes of administration are possible and dependent on the specific therapeutic application.

The present invention also presents a treatment for bone metastasis of prostata cancer by administering to a subject in need an effective amount of the modified hydroxypolymer conjugates

The targeting efficacy of the modified hydroxypolymer conjugate of the present invention, i.e. dextran-guanidine-biphosphonate conjugate was demonstrated with patients suffering from HRPC using radioactively labeled modified dextran-guanidine-biphosphonate. These Figures also demonstrate that the modified hydroxypolymer conjugates can be used for imaging and identification of tumor lesions in skeleton. The dextran-guanidine-biphosphonate can be radioactively labeled by a multitude of methods known in the art including the pertechnate -stannochloride method described in Example 2.

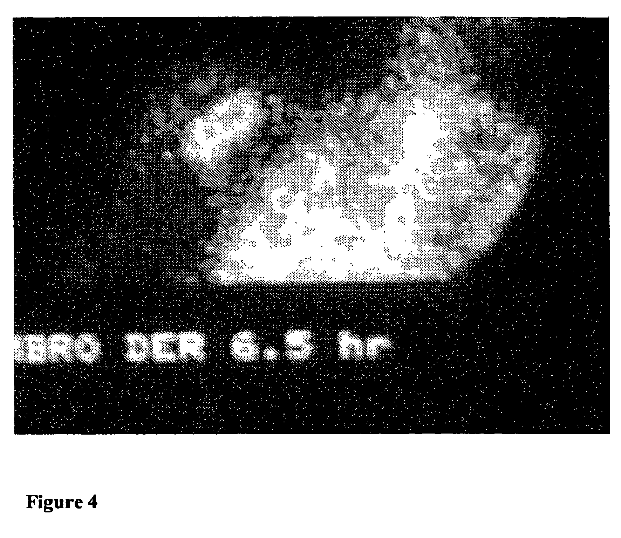

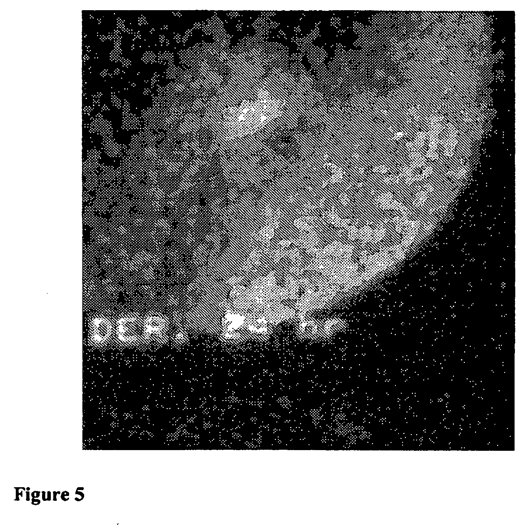

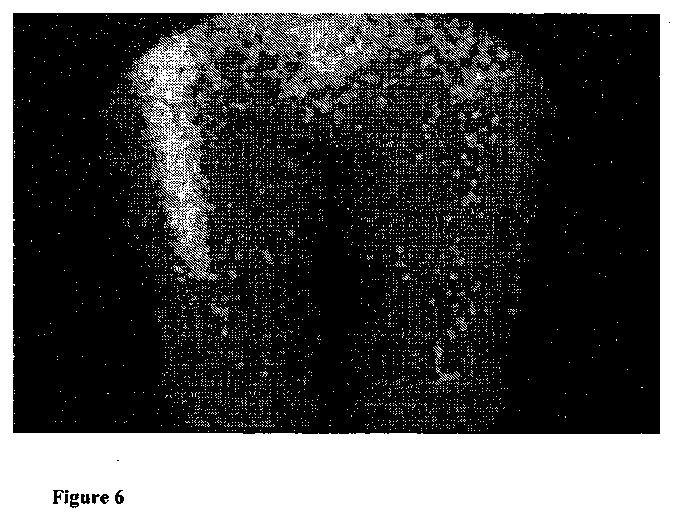

The results including clear uptake are shown in Figures 4 to 8 and demonstrate the targeting feasibility of the modified hydroxypolymer conjugate of present invention, i.e. dextran-guanidine-biphosphonate, and its potential for treatment of bone metastasis of prostate cancer. Simultaneously the results indicate that modified hydroxypolymer conjugate of the present invention, i.e. dextran-guanidine-biphosphonate, also has potential for the treatment of osteoporosis.

In the following examples the preparation of the compound and its effect are demonstrated.

### Example 1

### Synthesis of dextran-guanidine-biphosphonate

### Activation

Pharmaceutical grade dextran PM40 (150 mg) was dissolved in 5 mL of water. Sodium periodate 120 mg, was added, followed by 25 µL of concentrated sulphuric acid. The combined solution was stirred for 45 minutes. Ethylene glycol, 25 µl, was added in order to stop the oxidation and destroy excess periodate, by reacting for a further 15 minutes. The pH was then adjusted with 0.2 M Sodium Acetate to pH 6,5.

### Conjugation

2ml, obtained by the procedure above, was mixed with 20mg alendronate and sodium cyanoborohydride , 5 mg. The solution was stirred at room temperature and incubated for 1.5 hours. After 1.0 hours, 80mg aminoguanidine was added. The solutions was stirred for another 4 hours at room temperature and then separated from low-molecular components on PD 10 columns with PBS as eluent. 4 mL of purified conjugate solution was obtained.
Typically, the synthesis results in 20 - 30 mol of guanidine and 10- 20 mol of Alendronate per 1 mol of Dextran.

### Fluorometric Cytotoxicity Assay

The fluorometric cytotoxicity assay was performed as described by Larsson and Nygren (Larson et al, Anticancer Res, 1989, 9:1111-1119).

Briefly, approximately 10,000 cells/well were seeded (96-well microtiter plates, Falcon, Becton Dickinson, Meylan, France). Dextran-Guanidine-Biphosphonate (DGB) and in comparison, Zoledronic acid (Zometa®), at concentrations 0.4, 0.9, 1.75, 3.5, 7 and 25 µM were added to the wells. The control wells were given the same amount of PBS. After 72 h incubation the microtiter plates were centrifuged (200 x g for 3 minutes) and the medium was removed by flicking the plates. The cells were washed in PBS. Fluorescein diacetate (FDA, Sigma, Stockholm, Sweden) was dissolved in DMSO and kept frozen at -20°C as a stock solution (10 mg/ml). The FDA stock solution was diluted in PBS at a concentration of 10 µg/ml and 200 µl was added to each well. The plates were then incubated for 30 minutes at 37°C. A 96-well scanning fluorometer was used to count the emitted fluorescence from living cells. The data were transferred to a computer and the results were calculated.

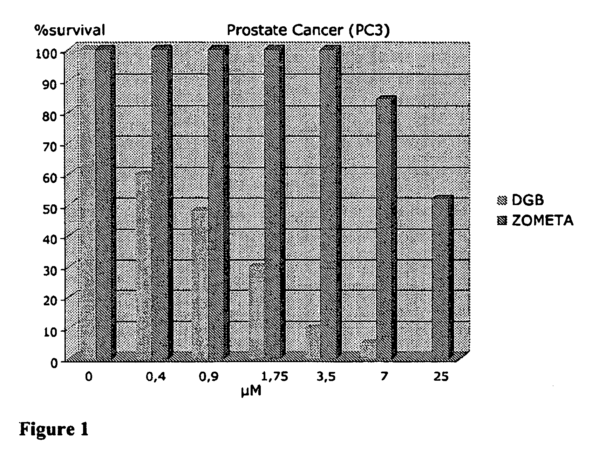

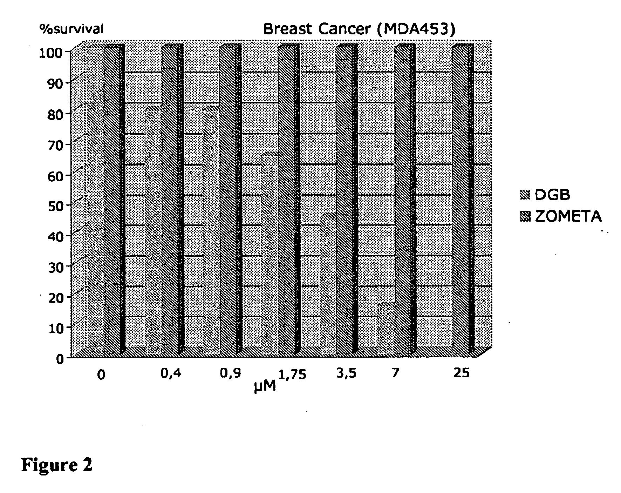

The results are shown in Figures 1 and 2, wherein the y axes show % cell survival and x axes show the molarity of the test substances. Two cell lines were used, PC3 (prostate cancer) and MDA453 (breast cancer). The results shown in Figure 1 indicate that the Dextran-Guanidine-Biphosphonate (DGB) in all concentrations used is more effective than the bone cancer medicine Zoledronic acid used for comparison. Complete killing of prostate cancer cells is achieved at a concentration of 25 µM, in which concentration Zoledronic acid has killed only half of the prostate cancer cells. The results shown in Figure 2 indicate that the Dextran-Guanidine-Biphosphonate (DGB) in all concentrations used is more effective than the bone cancer medicine Zoledronic acid used for comparison. Complete killing of the breast cancer cells is achieved at a concentration of 7 µM, in which concentration Zoledronic acid has almost no effect on the breast cancer cells.

### Example 2

### Synthesis of dextran-guanidine-biphosphonate

### Activation

Pharmaceutical grade dextran PM70 (150 mg) was dissolved in 6 mL of water. Sodium periodate 120 mg, was added, followed by 25 µL of concentrated sulphuric acid. The combined solution was stirred for 30 minutes. Ethylene glycol, 75 µl, was added in order to stop the oxidation and destroy excess periodate, by reacting for a further 15 minutes. The activated dextran was purified from low-molecular components by gel filtration in one mL portions on PD-10 columns with 0.1 M acetate, pH 6.5, as an eluent. In each separation, 2 mL of activated dextran solution was obtained.

### Conjugation mode 1

The eluent, 1ml, obtained by the procedure above, was mixed with 10mg alendronate and sodium cyanoborohydride, 5 mg. The solution was stirred at room temperature and incubated for 2 hours. After 2 hours, 65mg ²aminoguanidine was added. The solutions was stirred over night at room temperature and then separated from low-molecular components on PD 10 columns with PBS as eluent. Two mL of purified conjugate solution was obtained.

### Conjugation mode 2

The eluent, 1ml, obtained by the procedure above, was mixed with 65 mg aminoguanidine and sodium cyanoborohydride, 5 mg. The solution was stirred at room temperature and incubated for 2 hours. After 2 hours, 10 mg alendronate was added. The solutions was stirred over night at room temperature and then separated from low-molecular components on PD 10 columns with PBS as eluent. Two mL of purified conjugate solution was obtained.

### Radiolabelling

3mg of Guanidine-Dextran-Biphosphonate conjugate in 300 µl of 0,2M sodium acetate buffer at pH 6 was mixed with 300 µl pertechnetate, -700 MBq, and subsequently 10µl of stannous chloride (∼50µg in ethanol) was added and the solution was gently mixed and incubated for 15min. After incubation the solution was purified on a disposable sephadex G25 column (PD10). Finally the purified solution was filtered through a sterile filter (0,22 µ) and the resulting labelling yield was measured.

### Proof of the targeting concept:

Two hormone refractory prostate cancer patients with diagnosed bone lesions were injected with -3mg dextran-guanidine-biphosphonate labelled with -500 MBq Technetium-99m. Gamma images were collected after 2, 4, 6, 24 hours post injection.

### Result

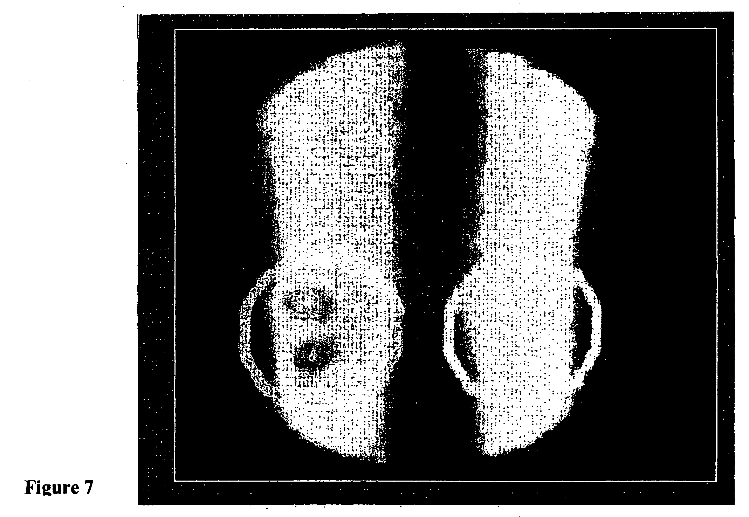

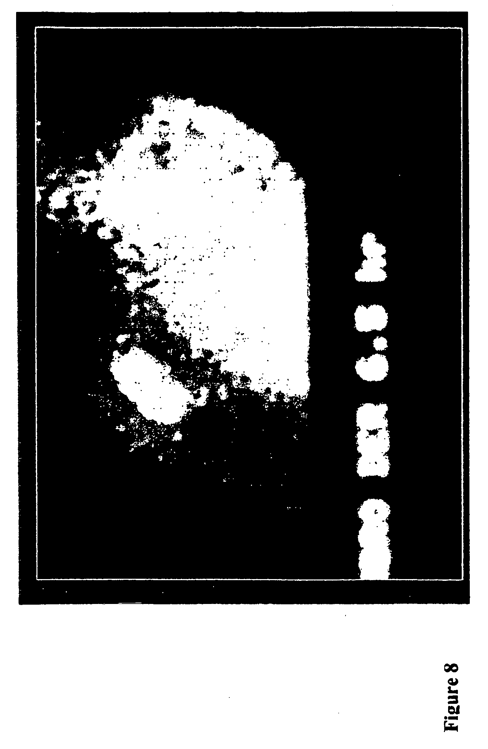

Clear uptake shown in the right knee (Figure 7) and the right shoulder (Figure 8). The images are in concordance with the result of the conventional bone-scan performed at diagnosis. The result demonstrates the feasibility of dextran-guanidine-biphosphonate and its potential for treatment of bone metastasis of prostate cancer. Dextran-guanidine-biphosphonate has potential for the treatment of osteoporosis.

## Claims

1. A modified hydroxypolymer conjugate, **characterized in that** the modified hydroxypolymer conjugate is a hydroxypolymer dextran substituted with a biphosphonate group and a guanidine group agmatine or aminoguanidine, which has at least one free amino group and which biphosphonate and guanidine groups are covalently coupled to activated hydroxyl groups of said hydroxypolymer, said conjugate having tumor cell killing effect.

2. The modified hydroxypolymer conjugate according to claim 1, **characterized in that** said biphosphonate group is neridronate or alendronate.

3. The modified hydroxypolymer conjugate according to claim 1, **characterized in that** said hydroxypolymer dextran has a molecular weight of 10³-10⁶ Daltons.

4. The modified hydroxypolymer conjugate according to claim 1, **characterized in that** the modified hydroxypolymer conjugate comprises at least 25 mol, preferably at least 30 mol, most preferably 35 mol guanidine groups agmatine or aminoguanidine per mol hydroxypolymer dextran and at least 10 mol, preferably at least 20 mol, most preferably 25 mol biphosphonate groups neridronate or alendronate per mol hydroxypolymer dextran.

5. The modified hydroxypolymer conjugate according to claim 1, **characterized in that** the modified hydroxypolymer conjugate comprises at least one additional covalently coupled functional group.

6. The modified hydroxypolymer conjugate according to claim 5, **characterized in that** said functional group is a radio-nuclide, a therapeutic compound, anticancer drug or a targeting agent.

7. The modified hydroxypolymer conjugate according to claim 1 for treating skeletal tumors or bone metastasis or osteoporosis.

8. The modified hydroxypolymer conjugate according to claim 1, **characterized in that** said modified hydroxypolymer conjugate is locally, intravesically, regiolocally, peritoneally, intratumorally, systemically or intravenously administrable.

9. The modified hydroxypolymer conjugate according to claim 1 for use in the treatment of skeletal tumors, bone metastasis or osteoporosis.

10. The use of modified hydroxypolymer conjugate according to claim 1 for manufacturing a medicine for treatment of skeletal tumors or bone metastasis or osteoporosis.

11. A composition having tumor cell killing effect, **characterized in that** said composition comprises the modified hydroxypolymer conjugate according to any of claims 1-9 and at least one pharmaceutically acceptable adjuvant.

12. A method for producing the modified hydroxypolymer conjugate according to claim 1, **characterized in that** a guanidine compound agmatine or aminoguanidine having at least one free amino group and a biphosphonate compound are sequentially added in an optional order into a solution comprising a purified activated hydroxypolymer dextran.

13. The method according to claim 12, **characterized in that** the activated hydroxypolymer is obtained by an oxidative reaction, wherein a periodate salt is added to an aqueous solution comprising a hydoxypolymer with subsequent addition of concentrated sulphuric acid and wherein the oxidative reaction is stopped by adding ethylene glycol, followed by purification with gel filtration.

14. The modified hydroxypolymer conjugate according to claim 9, **characterized in that** the skeletal tumors or bone metastasis are related to hormone refractory prostata cancer or breast cancer.

## Patentansprüche

1. Modifiziertes Hydroxypolymer-Konjugat, **dadurch gekennzeichnet, dass** das modifizierte Hydroxypolymer-Konjugat ein Hydroxypolymer-Dextran ist, das mit einer Biphosphonatgruppe und einer Guanidingruppe Agmatin oder Aminoguanidin substituiert ist, die mindestens eine freie Aminogruppe aufweist, und wobei die Biphosphonatgruppe und die Guanidingruppe kovalent an aktivierte Hydroxylgruppen des Hydroxypolymers gekuppelt sind, wobei das Konjugat eine Tumorzellen abtötende Wirkung hat.

2. Modifiziertes Hydroxpolymer-Konjugat nach Anspruch 1, **dadurch gekennzeichnet, dass** die Biphosphonatgruppe Neridronat oder Alendronat ist.

3. Modifiziertes Hydroxpolymer-Konjugat nach Anspruch 1, **dadurch gekennzeichnet, dass** das Hydroxypolymer-Dextran ein Molekulargewicht von 10³ - 10⁶ Dalton aufweist.

4. Modifiziertes Hydroxpolymer-Konjugat nach Anspruch 1, **dadurch gekennzeichnet, dass** das modifizierte Hydroxypolymer-Konjugat mindestens 25 Mol, vorzugsweise mindestens 30 Mol, am meisten bevorzugt 35 Mol Guanidingruppen Agmatin oder Aminoguanidin pro Mol Hydroxypolymer-Dextran und mindestens 10 Mol, vorzugsweise mindestens 20 Mol, am meisten bevorzugt 25 Mol Bisphosphonatgruppe Neridronat oder Alendronat umfasst.

5. Modifiziertes Hydroxpolymer-Konjugat nach Anspruch 1, **dadurch gekennzeichnet, dass** das modifizierte Hydroxypolymer-Konjugat mindestens eine zusätzliche kovalent gekuppelte funktionelle Gruppe umfasst.

6. Modifiziertes Hydroxypolymer-Konjugat nach Anspruch 5, **dadurch gekennzeichnet, dass** die funktionelle Gruppe ein Radionuklid, eine therapeutische Verbindung, eine antineoplastische Substanz oder ein Targeting-Mittel ist.

7. Modifiziertes Hydroxpolymer-Konjugat nach Anspruch 1 zur Behandlung von Skeletttumoren oder einer Knochenmetastase oder von Osteoporose.

8. Modifiziertes Hydroxpolymer-Konjugat nach Anspruch 1, **dadurch gekennzeichnet, dass** das modifizierte Hydroxypolymer-Konjugat lokal, intravesikal, regiolokal, peritoneal, intratumoral, systemisch oder intravenös verabreichbar ist.

9. Modifiziertes Hydroxpolymer-Konjugat nach Anspruch 1 zur Verwendung bei der Behandlung von Skeletttumoren, einer Knochenmetastase oder von Osteoporose.

10. Verwendung eines modifizierten Hydroxypolymer-Konjugats nach Anspruch 1 zur Herstellung eines Arzneimittels zur Behandlung von Skeletttumoren oder einer Knochenmetastase oder von Osteoporose.

11. Zusammensetzung mit einer Tumorzellen abtötenden Wirkung, **dadurch gekennzeichnet, dass** die Zusammensetzung das modifizierte Hydroxypolymer-Konjugat nach einem der Ansprüche 1 - 9 und mindestens einen pharmazeutisch unbedenklichen Hilfsstoff umfasst.

12. Verfahren zur Herstellung des modifizierten Hydroxypolymer-Konjugat nach Anspruch 1, **dadurch gekennzeichnet, dass** eine Guanidinverbindung Agmatin oder Aminoguanidin mit mindestens einer freien Aminogruppe und eine Biphosphonatgruppe nacheinander in einer optionalen Reihenfolge in eine Lösung gegeben werden, die ein aufgereinigtes aktiviertes Hydroxypolymer-Dextran umfasst.

13. Verfahren nach Anspruch 12, **dadurch gekennzeichnet, dass** das aktivierte Hydroxypolymer durch eine oxidative Reaktion erhalten wird, wobei ein Periodatsalz einer wässrigen Lösung zugegeben wird, die ein Hydroxypolymer umfasst, mit anschließender Zugabe von konzentrierter Schwefelsäure, und wobei die oxidative Reaktion durch Zugeben von Ethylenglykol angehalten wird, gefolgt von Aufreinigung mit Gelfiltration.

14. Modifiziertes Hydroxypolymer-Konjugat nach Anspruch 9, **dadurch gekennzeichnet, dass** die Skeletttumoren oder die Knochenmetastase mit hormonresistentem Prostatakrebs oder Brustkrebs in Verbindung stehen bzw. steht.

## Revendications

1. Conjugué hydroxypolymère modifié, **caractérisé en ce que** le conjugué hydroxypolymère modifié est un dextrane hydroxypolymère substitué avec un groupe biphosphonate et un groupe guanidine agmatine ou aminoguanidine, qui a au moins un groupe aminé libre et lesquels groupes biphosphonate et guanidine sont couplés de manière covalente à des groupes hydroxyle activés, dudit hydroxypolymère, ledit conjugué ayant un effet de destruction des cellules tumorales.

2. Conjugué hydroxypolymère modifié selon la revendication 1, **caractérisé en ce que** ledit groupe biphosphonate est le néridronate ou l'alendronate.

3. Conjugué hydroxypolymère modifié selon revendication 1, **caractérisé en ce que** ledit dextrane hydroxypolymère a un poids moléculaire de 10³ à 10⁶ Daltons.

4. Conjugué hydroxypolymère modifié selon la revendication 1, **caractérisé en ce que** le conjugué hydroxypolymère comprend au moins 25 mol, de préférence au moins 30 mol, de manière la plus appréciée 35 mol de groupes guanidine agmatine ou aminoguanidine par mole de dextrane hydroxypolymère et au moins 10 mol, de préférence au moins 20 mol, de manière préférée entre toutes 25 mol de groupes biphosphonate néridronate ou alendronate par mole de dextrane hydroxypolymère.

5. Conjugué hydroxypolymère modifié selon la revendication 1, **caractérisé en ce que** le conjugué hydroxypolymère modifié comprend au moins un groupe fonctionnel supplémentaire couplé de manière covalente.

6. Conjugué hydroxypolymère modifié selon la revendication 5, **caractérisé en ce que** ledit groupe fonctionnel est un radionucléide, un composé thérapeutique, un médicament anticancéreux ou un agent de ciblage.

7. Conjugué hydroxypolymère modifié selon la revendication 1 pour le traitement de tumeurs osseuses ou des métastases osseuses ou de l'ostéoporose.

8. Conjugué hydroxypolymère modifié selon la revendication 1, **caractérisé en ce que** ledit conjugué hydroxypolymère modifié peut être administré par voie locale, intravésicale, régiolocale, péritonéale, intratumorale, systémique ou intraveineuse.

9. Conjugué hydroxypolymère modifié selon la revendication 1 pour l'utilisation dans le traitement des tumeurs osseuses, des métastases osseuses ou de l'ostéoporose.

10. Utilisation du conjugué hydroxypolymère modifié selon la revendication 1 pour la fabrication d'un médicament pour le traitement des tumeurs osseuses ou des métastases osseuses ou de l'ostéoporose.

11. Composition ayant un effet de destruction des cellules tumorales, **caractérisée en ce que** ladite composition comprend le conjugué hydroxypolymère modifié selon l'une quelconque des revendications 1 à 9 et au moins un adjuvant pharmaceutiquement acceptable.

12. Procédé de production du conjugué hydroxypolymère modifié selon la revendication 1, **caractérisé en ce qu'**un composé guanidine agmatine ou aminoguanidine ayant au moins un groupe amino libre et un composé biphosphonate sont ajoutés de manière séquentielle selon un ordre facultatif dans une solution comprenant un dextrane hydroxypolymère activé purifié.

13. Procédé selon la revendication 12, **caractérisé en ce que** l'hydroxypolymère activé est obtenu par une réaction d'oxydation, dans laquelle un sel périodate est ajouté à une solution aqueuse comprenant un hydroxypolymère avec addition ultérieure d'acide sulfurique concentré et dans lequel la réaction d'oxydation est stoppée par l'addition d'éthylène glycol, suivie de la purification avec filtration sur gel.

14. Conjugué hydroxypolymère modifié selon la revendication 9, **caractérisé en ce que** les tumeurs osseuses ou les métastases osseuses sont liées à un cancer de la prostate réfractaire aux hormones ou un cancer du sein.
